# FASCICULE DE BREVET EUROPEEN

(11) **EP 3 471 557 B1**
(45) Date de publication et mention de la délivrance du brevet: **02.07.2025**
(21) Numéro de dépôt: 17732392.0
(22) Date de dépôt: 20.06.2017
(51) Int. Cl.: A23B 7/144, B01D 1/00, B01D 47/02, B01D 53/14, A61L 9/14

(54) **PROCÉDÉ ET ENSEMBLE DE TRAITEMENT DE L'ATMOSPHÈRE D'UN STOCKAGE DE PRODUITS VÉGÉTAUX**
VERFAHREN UND VORRICHTUNG ZUR BEHANDLUNG DER LAGERUNGSATMOSPHÄRE VON PFLANZENERZEUGNISSEN
PROCESS AND SET FOR TREATING THE STORAGE ATMOSPHERE OF PLANT PRODUCTS

(30) Priorité: 20.06.2016 FR 1655717; 30.08.2016 FR 1658046
(43) Date de publication de la demande: 24.04.2019
(73) Titulaire: Xeda International S.A., 13670 Saint Andiol (FR)
(72) Inventeur: SARDO, Alberto, 13160 Chateaunard (FR); SARDO, Stéfano, 13160 Chateaunard (FR)
(74) Mandataire: Lavoix
(86) Numéro de dépôt international: PCT/EP2017/065102
(87) Numéro de publication internationale: WO 2017/220587

(56) Documents cités:
- EP-A1- 2 918 336
- WO-A1-00/32063
- WO-A1-2012/081015
- WO-A1-2017/042072
- WO-A2-2007/026363
- FR-A- 1 338 080
- FR-A1- 2 441 812
- GB-A- 476 272
- GB-A- 782 848
- US-A- 5 451 248
- US-B1- 7 638 114

## Description

L'invention concerne en général la conservation des produits végétaux stockés dans des chambres de grand volume.

Plus précisément, l'invention concerne selon un premier aspect un procédé de traitement de l'atmosphère d'un stockage de produits végétaux ayant un volume supérieur à 1000 m³.

Il est nécessaire de maîtriser un grand nombre de paramètres pour contrôler le processus de maturation des produits végétaux stockés.

La respiration des produits végétaux conservés dépend de la variété végétale, de leur état de maturation, de la température de stockage et de la composition de l'atmosphère.

Les produits végétaux absorbent l'oxygène et produisent du gaz carbonique, des arômes et de l'éthylène. Par une réaction en chaîne, l'éthylène produit accélère le processus de maturation. La concentration de ces différents gaz dans l'atmosphère doit être contrôlée.

Par ailleurs, il est nécessaire de maintenir une humidité suffisante à l'intérieur du stockage.

De plus, les produits végétaux sont typiquement traités en injectant dans l'atmosphère des produits phytoprotecteurs, qui sont prévus pour améliorer la conservation des produits végétaux. US 7 638 114 décrit la désinfection d'atmosphère en particulier dans le domaine alimentaire par évaporation d'actifs, de préférence par nébulisation.

Dans ce contexte, l'invention vise à proposer un procédé qui permette d'assurer une ou plusieurs des fonctions ci-dessus, de manière efficace.

A cette fin, l'invention porte sur un procédé de traitement de l'atmosphère d'un stockage de produits végétaux ayant un volume supérieur à 1000 m³, le procédé comprenant au moins une étape de mise en contact de l'atmosphère avec un flux de liquide par circulation dans un garnissage.

Le procédé peut en outre présenter une ou plusieurs des caractéristiques ci-dessous, considérées individuellement ou selon toutes les combinaisons techniquement possibles :
- le liquide comprend au moins un produit biocide et/ou phytoprotecteur volatile, de température d'ébullition comprise entre 60 et 280°C, le liquide étant évaporé à l'étape de mise en contact à une température inférieure à 50°C ; (cette caractéristique est essentielle à l'invention)
- le liquide est de l'eau, l'étape de mise en contact visant à éliminer les poussières et les arômes de l'atmosphère ;
- le liquide contient au moins un additif, l'étape de mise en contact visant à éliminer le CO₂ et/ou l'éthylène de l'atmosphère ;
- le au moins un additif comprend un oxydant, par exemple du permanganate ou du bichromate ;
- le procédé comprend, après l'étape de mise en contact, une autre étape de mise en contact de l'atmosphère avec au moins un flux d'un autre liquide par circulation dans un autre garnissage, l'autre liquide comprenant au moins un produit biocide et/ou phytoprotecteur volatile, de température d'ébullition comprise entre 60 et 280°C, l'autre liquide étant évaporé à l'autre étape de mise en contact à une température inférieure à 50°C ;
- le procédé comprend une étape de mesure de la concentration du produit biocide et/ou phytoprotecteur volatile dans l'atmosphère, un débit du flux de l'autre liquide à l'étape de mise en contact étant ajusté en fonction de ladite concentration mesurée ;
- le procédé comprend :
   - une étape de collecte du flux de liquide issu de l'étape de mise en contact ;
   - une étape de régénération du flux de liquide issu de l'étape de mise en contact, au cours de laquelle ledit flux de liquide est mis en contact avec un flux d'air extérieur au stockage par circulation dans un garnissage extérieur, le flux de liquide étant ensuite réutilisé à l'étape de mise en contact ;
- le procédé comprend une étape de mesure de la concentration en CO₂ et/ou O₂ dans l'atmosphère du stockage, un débit du flux de liquide étant réglé en fonction de ladite concentration mesurée ;
- le procédé comprend une étape de mesure de la température et/ou de l'humidité dans l'atmosphère du stockage, un débit de l'atmosphère à l'étape de mise en contact étant réglé en fonction de ladite température et/ou humidité mesurée ; et
- le procédé comprend une étape de mesure de la température dans l'atmosphère du stockage, un débit du flux d'air extérieur à l'étape de régénération étant réglé au moins en fonction de ladite température mesurée.

Selon un second aspect, l'invention porte sur un ensemble de traitement de l'atmosphère d'un stockage de produits végétaux ayant un volume supérieur à 1000 m³, l'ensemble comprenant au moins :
- un dispositif de mise en contact, comportant un garnissage ;
- un dispositif d'injection d'un flux de liquide dans le dispositif de mise en contact ;
- un dispositif de circulation de l'atmosphère du stockage dans le dispositif de mise en contact ;
le dispositif de mise en contact étant conformé pour que l'atmosphère soit mise en contact avec le flux de liquide par circulation dans le garnissage.

Selon un troisième aspect, l'invention porte sur un système comprenant un stockage de produits végétaux ayant un volume supérieur à 1000 m³, et un ensemble de traitement de l'atmosphère dudit stockage ayant les caractéristiques ci-dessus. Le stockage contient avantageusement une quantité de produits végétaux.

L'ensemble peut en outre présenter une ou plusieurs des caractéristiques ci-dessous, considérées individuellement ou selon toutes les combinaisons techniquement possibles :
- le dispositif de mise en contact comporte une réserve et une dose de liquide stockée dans la réserve, le liquide comprenant au moins un produit biocide et/ou phytoprotecteur volatile, de température d'ébullition comprise entre 60 et 280°C, le liquide étant évaporé dans le dispositif de mise en contact à une température inférieure à 50°C ; (cette caractéristique est essentielle à l'invention)
- le liquide est de l'eau, le dispositif de mise en contact étant configuré pour éliminer les poussières et les arômes de l'atmosphère ;
- le liquide contient au moins un additif, le dispositif de de mise en contact étant configuré pour éliminer le CO₂ et/ou l'éthylène de l'atmosphère ;
- l'ensemble comprend :
   - un autre dispositif de mise en contact, comportant un autre garnissage ;
   - un dispositif d'injection d'un flux d'un autre liquide dans l'autre dispositif de mise en contact ;
   - un dispositif configuré pour faire circuler l'atmosphère sortant du dispositif de mise en contact dans l'autre dispositif de mise en contact ;

l'autre dispositif de mise en contact étant conformé pour que l'atmosphère soit mise en contact avec l'autre flux de liquide par circulation dans l'autre garnissage ; l'autre dispositif de mise en contact comporte une autre réserve et une dose de l'autre liquide stockée dans l'autre réserve, l'autre liquide comprenant au moins un produit biocide et/ou phytoprotecteur volatile, de température d'ébullition comprise entre 60 et 280°C, l'autre liquide étant évaporé dans l'autre dispositif de mise en contact à une température inférieure à 50°C ;
   - l'ensemble comprend un dispositif de régénération du flux de liquide, avec :
      - un dispositif extérieur de mise en contact, comportant un garnissage extérieur ;
      - un dispositif d'injection du flux de liquide provenant du dispositif de mise en contact dans le dispositif extérieur de mise en contact ;
      - un dispositif de circulation d'un flux d'air extérieur au stockage (3) dans le dispositif extérieur de mise en contact ; et
le dispositif extérieur de mise en contact étant conformé pour que le flux de liquide soit mis en contact avec un flux d'air extérieur au stockage par circulation dans le garnissage extérieur, puis recyclé dans le dispositif de mise en contact.

D'autres caractéristiques et avantages de l'invention ressortiront de la description détaillée qui en est donnée ci-dessous, à titre indicatif et nullement limitatif, en référence aux figures annexées, parmi lesquelles :
- la figure 1 est une représentation schématique simplifiée d'un premier mode de réalisation de l'invention ;
- la figure 2 est une représentation schématique simplifiée d'un second mode de réalisation de l'invention ;
- la figure 3 représente une variante du second mode de réalisation de l'invention ;
- la figure 4 est une représentation schématique simplifiée d'un troisième mode de réalisation de l'invention ; et
- la figure 5 est une représentation schématique simplifiée d'un quatrième mode de réalisation de l'invention.

L'ensemble 1 illustré sur les figures, et le procédé correspondant, est destiné au traitement de l'atmosphère d'un stockage 3 de produits végétaux 5 ayant un volume supérieur à 1000 m³.

Ainsi, l'ensemble et le procédé sont destinés à être appliqués à des stockages de grand volume, par exemple une chambre, un silo, une serre ou tout autre local destiné au stockage de produits végétaux. Le stockage est une enceinte fermée, au sens où les échanges entre l'atmosphère du stockage et l'extérieur, notamment les échanges gazeux sont très réduits, de manière à ne pas mettre en péril la conservation des produits végétaux.

Les produits végétaux sont typiquement des fruits ou des légumes, qui sont conservés après récolte et avant expédition vers les détaillants.

Le volume du stockage est supérieur à 1000 m³.

L'ensemble 1 comprend au moins :
- un dispositif de mise en contact, comportant un garnissage ;
- un organe d'injection d'un flux de liquide dans le dispositif de mise en contact ;
- un organe de circulation de l'atmosphère du stockage dans le dispositif de mise en contact.

Le dispositif de mise en contact est conformé pour que l'atmosphère soit mise en contact avec le flux de liquide par circulation dans le garnissage.

Le procédé correspondant comprend au moins une étape de mise en contact de l'atmosphère avec un flux de liquide par circulation dans un garnissage.

La mise en contact est effectuée dans tout type de dispositif contenant le garnissage. Par exemple, elle est effectuée dans une tour à garnissage.

On entend ici par garnissage tout type de structure permettant d'obtenir une surface de contact importante entre une phase liquide et une phase gazeuse, et ainsi d'améliorer les échanges entre la phase liquide et la phase gazeuse.

Le garnissage peut ainsi être un garnissage de type vrac ou un garnissage de type structuré.

Dans le cas présent, le garnissage est par exemple du type anneau de Raschig ou anneau de Pall, ou encore est un garnissage structuré en nids d'abeilles.

Il est typiquement réalisé dans une matière plastique.

La mise en contact permet de réaliser des transferts particulièrement efficaces entre le flux de liquide et l'atmosphère du stockage.

Comme il sera décrit ci-dessous, ces transferts visent différents buts, en fonction de la nature du liquide :
- filtration de l'atmosphère, en particulier piégeage de la poussière et des terres en suspension dans l'atmosphère ;
- humidification de l'atmosphère ;
- élimination du CO₂ dégagé par les produits végétaux ;
- élimination de l'éthylène (C₂H₄) dégagé par les produits végétaux ;
- élimination des arômes dégagés par les produits végétaux ;
- réintégration de l'oxygène consommée par les produits végétaux ;
- stérilisation de l'atmosphère ;
- application d'un traitement de protection des produits végétaux.

L'atmosphère du stockage correspond ici au volume des gaz remplissant le stockage et baignant les produits végétaux.

Cette atmosphère comprend typiquement de l'air, plus les gaz et les produits dégagés par les produits végétaux au cours de leur maturation. Elle comprend également de la vapeur d'eau.

En variante, l'atmosphère est une atmosphère modifiée, par exemple appauvrie en oxygène. Ceci est le cas notamment pour le stockage de certains produits végétaux comme les pommes.

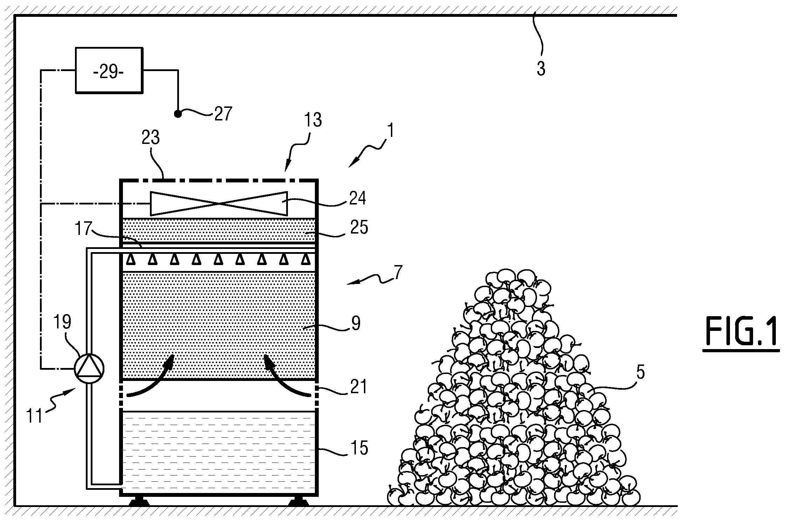

Comme illustré sur la figure 1, le liquide utilisé comprend au moins un produit biocide et/ou phytoprotecteur volatile, de température d'ébullition comprise entre 60 et 280°C, le liquide étant évaporé à l'étape de mise en contact à une température inférieure à 50°C.

Quand le produit est un produit biocide, le traitement vise à assainir le stockage. Typiquement, ce traitement est appliqué quand le stockage ne contient pas de produits végétaux.

Quand le produit est un produit phytoprotecteur, qui peut aussi être appelé produit phytosanitaire, le traitement vise à protéger les produits végétaux, en empêchant le développement de maladies et/ou des pourritures.

Le liquide ne contient que des produits biocides, ou que des produits phytosanitaires, ou encore comprend un ou plusieurs produits biocides mélangés à un ou plusieurs produits phytosanitaires.

Au moins un des produits phytosanitaire est choisi dans la liste suivante : huile essentielle, terpènes, alcool de C3 à C9 saturé ou insaturé, comme par exemple l'isopropanol, l'iso-octanol, le 2-éthylhexanol, les produits de synthèse volatiles, comme par exemple le glutaraldéhyde, l'hexanal, le diméthylnaphtalène et le 3-décène-2-one.

L'huile essentielle est par exemple choisie dans le groupe formé par l'huile de menthe, l'huile de girofle, l'huile de rose, l'huile de thym, l'huile d'origan. En variante, le liquide comprend l'un des constituants de ces huiles, choisi dans l'ensemble formé par le L-carvone, l'eugénol, le géraniol, le thymol, le carvacrol.

Pour une application de désinfection, le produit biocide est un produit volatil, naturel ou de synthèse, ayant des propriétés biocides, tel que l'huile de girofle, l'huile de thym, le geraniol, l'alcool ethylique, le glutaraldéhyde.

Typiquement, le liquide comprend seulement le ou les produits, sans solvant ni adjuvant. En variante, le liquide comporte un solvant aqueux ou organique, dans lequel est dissout le ou les produits et un ou plusieurs adjuvants. Le solvant aqueux est par exemple de l'eau. Le solvant organique est par exemple un solvant du type décrit dans FR 2 791 910 ou des glycols, di-glycols et leurs esters relatifs. Les adjuvants sont par exemple des substances aptes à véhiculer la ou les matières actives ou aptes à donner un effet de dilution.

En tout état de cause, le liquide au cours de l'étape de mise en contact est vaporisé à une température inférieure à 50°C, de préférence inférieure à 20°C, notamment comprise entre -2°C et +12°C, et en particulier entre 0 et 10°C. Par exemple, le liquide est évaporé à température ambiante.

Comme illustré sur la figure 1, l'ensemble de traitement 1 comprend:
- le dispositif 7 de mise en contact, comportant un garnissage 9 ;
- le dispositif 11 d'injection du flux de liquide dans le dispositif de mise en contact 7 ;
- le dispositif 13 de circulation de l'atmosphère du stockage dans le dispositif de mise en contact 7.

Le dispositif de mise en contact 7 est conformé pour que l'atmosphère soit mise en contact avec le flux de liquide par circulation dans le garnissage 9. Typiquement, le dispositif de mise en contact 7 est une tour à garnissage, qui est d'axe vertical dans l'exemple représenté.

Le dispositif d'injection 11 comporte une réserve 15 de liquide, et une dose de liquide stockée dans la réserve 15. La réserve 15 est typiquement un bac, placé verticalement sous le garnissage 9.

Le dispositif d'injection 11 est agencé pour injecter le liquide au-dessus du garnissage 9.

A cet effet, il comprend typiquement un ou plusieurs organes d'aspersion 17, par exemple des rampes, placés au-dessus du garnissage, et un organe de transfert 19, tel qu'une pompe, aspirant le liquide dans la réserve 15 et refoulant celui-ci dans le ou les organes 17.

Le dispositif de circulation 13 est agencé pour créer une circulation ascendante de l'atmosphère à l'intérieur du dispositif de mise en contact 7.

Pour ce faire, le dispositif de mise en contact 7 comporte une ou plusieurs entrées 21 pour l'atmosphère débouchant à l'intérieur du dispositif de mise en contact 7, sous le garnissage 9.

Chaque entrée 21 communique fluidiquement avec l'intérieur du stockage 3.

Le dispositif de mise en contact présente une sortie 23 pour l'atmosphère chargée en liquide évaporé, placée en partie supérieure du dispositif de mise en contact, au-dessus des garnissages 9. La sortie 23 est raccordée fluidiquement avec l'intérieur du stockage 3.

Le dispositif de circulation 13 comprend par exemple un organe de circulation 24 tel qu'un ventilateur ou une soufflante, placé au-dessus du garnissage 9, typiquement au sommet du dispositif de mise en contact 7.

L'organe de circulation 24 aspire l'atmosphère chargée en liquide évaporé au-dessus du garnissage 9, et le refoule dans ou vers la sortie 23.

Le garnissage 9, comme précisé plus haut, est de tout type adapté.

De préférence, le dispositif de mise en contact 1 comporte un séparateur de gouttes 25, placé au-dessus des organes d'aspersion 17, et plus précisément entre les organes d'aspersion 17 et l'organe de circulation 24.

Dans un exemple de réalisation, le dispositif de mise en contact 7 présente une section horizontale carrée, sensiblement constante, de 700x700 mm. La réserve 15 présente la même section horizontale, et présente une hauteur comprise entre 500 et 700 mm. Le dispositif présente quatre entrées 21, chacune disposée sur un des côtés. Le garnissage 9 présente une hauteur d'environ 1 m. Le garnissage est placé par exemple 700 mm au-dessous de l'arrivée de liquide, le séparateur de gouttes 25 étant placé 300 mm au-dessus de l'arrivée de liquide.

L'ensemble de traitement 1 comporte de préférence un capteur 27 de mesure de la concentration du produit biocide et/ou phytoprotecteur volatile dans l'atmosphère et un dispositif électronique 29 renseigné par le capteur 27.

Le dispositif électronique 29 est programmé pour piloter le dispositif d'injection 11 et/ou l'organe de circulation 13.

Plus précisément, il est notamment programmé pour régler le débit du flux de liquide en fonction de la concentration mesurée par le capteur 27. De préférence, il pilote également l'organe de circulation 24.

Le dispositif électronique 29 est par exemple un calculateur ou une partie de calculateur. En variante, le dispositif électronique de pilotage 29 est réalisé sous forme de composants logiques programmables (FPGA, Field Programmable Gate Array) ou sous forme d'un circuit intégré dédié (ASIC, Application Specific Integrated Circuit). Le dispositif électronique 29 est programmé pour mettre en œuvre une stratégie de traitement.

La stratégie de traitement peut être de tout type. Comme décrit dans la demande française déposée le même jour que la présente demande de brevet, le traitement peut s'étaler sur une longue durée, de manière à injecter le produit par petite quantité, progressivement, de manière à maintenir la concentration de produits à l'intérieur du stockage constamment à un niveau modéré.

Inversement, il est possible de réaliser un traitement visant à obtenir rapidement la concentration de saturation du produit dans l'atmosphère, sur une période courte. Ceci permet par exemple d'obtenir la stérilisation du stockage vide ou partiellement vide. Plutôt que maintenir dans ce cas une concentration de produits actifs moins élevée pendant une longue durée, on cherche au contraire à parfaitement stériliser le stockage en saturant l'atmosphère très rapidement, la vapeur du produit agissant ainsi de façon immédiate et optimale en tous points du local.

Cet effet peut être obtenu du fait que la surface de contact entre le liquide et l'atmosphère est élevée, du fait de la présence des garnissages. La machine vendue sous le nom de XEDAVAP^{®}, dont le principe est d'injecter le liquide à évaporer sur une toile balayée par un courant d'air, a une surface de toile développée maximum d'environ 4 m². Ceci permet d'évaporer par exemple 1,2 litre/jour d'huile de menthe.

Au contraire, l'ensemble de traitement de la présente invention offre une surface de contact entre le gaz et le liquide qui peut aller par exemple jusqu'à 70 m². On peut ainsi évaporer des quantités de produits beaucoup plus importantes, par exemple 20 litres de produit par jour, et atteindre plus rapidement la concentration de saturation du produit dans l'atmosphère.

Le fonctionnement de l'ensemble est le suivant.

Le liquide à évaporer est disposé dans la réserve 15. L'organe de transfert 19 refoule le liquide dans le ou les organes d'aspersion 17, qui projettent le liquide vers le garnissage 9. L'organe 24 de mise en circulation de l'atmosphère crée un flux gazeux ascendant. L'atmosphère pénètre dans le dispositif 7 par les entrées 21, circule vers le haut à travers le garnissage 9. Le liquide circule quant à lui vers le bas à travers le garnissage 9, une partie du liquide étant évaporée au contact du flux gazeux et étant entraînée avec l'atmosphère sous forme de vapeur. La fraction du liquide qui n'est pas évaporée retombe dans la réserve 15. Elle est recyclée. L'atmosphère chargée en liquide évaporé passe à travers le séparateur de gouttes 25 et est refoulée par l'organe de circulation 24 jusqu'à la sortie 23.

L'ensemble 1 est typiquement placé à l'intérieur du stockage. Il aspire ainsi par la ou les entrées 21 directement l'atmosphère du stockage, et rejette cette atmosphère chargée en vapeur directement dans le stockage, par la sortie 23.

Le débit de liquide est par exemple de 3 m³/heure, et le débit de l'atmosphère d'environ 2000 m³/heure.

Le procédé de traitement selon le premier mode de réalisation comprend une étape de mise en contact de l'atmosphère du stockage 3 avec au moins un flux de liquide par circulation dans un autre garnissage, le liquide comprenant au moins un produit biocide et/ou phytoprotecteur volatile, de température d'ébullition comprise entre 60 et 280°C, le liquide étant évaporé à l'autre étape de mise en contact à une température inférieure à 50°C.

Typiquement, le procédé comprend une étape de mesure de la concentration du produit biocide et/ou phytoprotecteur volatile dans l'atmosphère, le débit du flux de liquide à l'étape de mise en contact étant ajusté en fonction de la concentration mesurée.

De préférence, le liquide injecté est collecté sous le garnissage, dans une réserve, et recyclé à l'étape de mise en contact.

Le procédé est prévu pour être mis en œuvre par l'ensemble de traitement 1 décrit ci-dessus. Inversement, l'ensemble de traitement 1 décrit ci-dessus est particulièrement adapté pour la mise en œuvre du procédé.

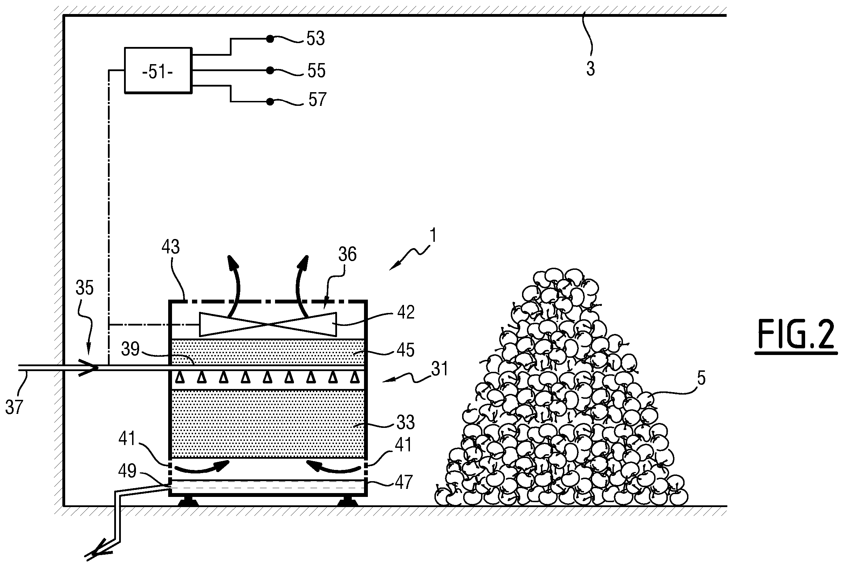

Un second mode de réalisation de l'invention va maintenant être décrit, en référence à la figure 2. Seuls les points par lesquels ce second mode de réalisation diffère du premier seront détaillés ci-dessous.

Dans le second mode de réalisation, le liquide est de l'eau, et la mise en contact vise à éliminer les poussières et les arômes de l'atmosphère. Les arômes sont dégagés par les produits végétaux pendant leur maturation.

L'ensemble 1 comprend comme dans le premier mode de réalisation :
- un dispositif 31 de mise en contact, comportant un garnissage 33 ;
- un dispositif 35 d'injection du flux de liquide dans le dispositif de mise en contact 31 ;
- un dispositif 36 de circulation de l'atmosphère dans le dispositif de mise en contact 31.

Le dispositif d'injection 35 comporte une arrivée d'eau 37, alimentant un ou plusieurs organes d'aspersion 39 tels que des rampes. L'arrivée d'eau 35 est typiquement raccordée à un réseau de distribution d'eau, ou à un réservoir d'eau. L'eau est typiquement de l'eau pure, ne comportant pas d'additif. L'eau est à une température inférieure à 50°C, typiquement à température ambiante.

Le dispositif de circulation 36 est agencé pour créer une circulation ascendante de l'atmosphère à l'intérieur du dispositif de mise en contact 31.

Pour ce faire, le dispositif de mise en contact 31 comporte une ou plusieurs entrées 41 pour l'atmosphère débouchant à l'intérieur du dispositif de mise en contact 31, sous le garnissage 33. Le dispositif de circulation 36 comporte un organe de circulation 42 est par exemple un ventilateur ou une soufflante, placé(e) au-dessus du garnissage 33, refoulant l'atmosphère dans une sortie 43 communiquant fluidiquement avec l'intérieur du stockage. Un séparateur de gouttes 45 est interposé verticalement entre le ou les organes d'aspersion 39 et l'organe de circulation 42.

Le dispositif de mise en contact 35 comporte par ailleurs un bac 47 placé sous le garnissage 33, et prévu pour collecter l'eau qui n'est pas évaporée au contact de l'atmosphère dans le garnissage 33. Le bac de collecte 47 présente une sortie 49 raccordée typiquement à un égout.

Ainsi, l'eau usée contenant les produits indésirables, c'est-à-dire au moins les poussières et les arômes, est déchargée du dispositif de mise en contact 7 vers les égouts. Elle n'est pas recyclée.

En variante, l'eau est recyclée après avoir été purifiée.

Comme dans le premier mode de réalisation, un dispositif électronique 51 pilote l'organe de circulation 42 et le dispositif d'injection 35.

Typiquement, l'ensemble de traitement est mis en fonctionnement périodiquement, par exemple une fois par jour, de manière à assainir l'atmosphère du stockage.

Le débit d'eau est typiquement compris entre 300 et 500 litres/heure, le débit de l'atmosphère à travers le garnissage étant de l'ordre de 2000 m³/heure.

Il est à noter que dispositif et le procédé selon le second mode de réalisation, outre le piégeage de la poussière et l'élimination des arômes, permettent d'humidifier l'atmosphère du stockage 3, et également, le cas échéant, d'enrichir l'atmosphère en oxygène.

En effet, comme précisé plus haut, il est nécessaire généralement de maintenir un certain taux d'humidité dans l'atmosphère du stockage, pour éviter que les produits végétaux ne dessèchent, et présentent un aspect ridé.

Quand le stockage est équipé d'un dispositif de conditionnement d'air, destiné à maintenir la température à l'intérieur du stockage dans une fourchette prédéterminée, il se produit une condensation de la vapeur d'eau contenue dans l'atmosphère notamment au niveau des échangeurs de chaleur ou des condenseurs du dispositif de conditionnement d'air.

Le liquide circulant dans le dispositif de mise en contact est partiellement évaporé, ce qui compense au moins partiellement la vapeur d'eau condensée dans le dispositif de conditionnement d'air.

Par ailleurs, les produits végétaux stockés dans le stockage consomment l'oxygène de l'atmosphère par leur respiration naturelle. Il est donc nécessaire de réenrichir régulièrement l'atmosphère en oxygène. Dans le cas présent, en particulier dans le second mode de réalisation, l'eau alimentant le dispositif de mise en contact contient de l'oxygène dissous, qui est partiellement vaporisée lors de la mise en contact avec l'atmosphère.

De préférence, l'ensemble 1 comporte un capteur 53 de mesure de la température à l'intérieur du stockage 3. Le dispositif électronique 51 est configuré pour régler le débit du flux de liquide et/ou le débit de l'atmosphère au moins en fonction de la température mesurée.

Avantageusement, l'ensemble 1 comporte un capteur 55, configuré pour analyser la concentration en O₂ dans l'atmosphère du stockage, typiquement un analyseur de gaz. Le dispositif électronique 51 est configuré pour régler le débit du flux de liquide au moins en fonction de la concentration mesurée.

Par exemple, l'ensemble 1 comporte un hygromètre 57, agencé pour mesurer l'humidité de l'atmosphère dans la chambre 3. Le dispositif électronique 51 est programmé pour régler le débit de l'atmosphère et/ou le débit du flux de liquide au moins en fonction de l'humidité mesurée par l'hygromètre 57.

Selon une variante du second mode de réalisation, le liquide mis en contact avec l'atmosphère du stockage contient au moins un additif, le dispositif de mise en contact étant configuré pour éliminer le CO₂ et/ou l'éthylène de l'atmosphère du stockage.

Ainsi, le liquide comprend de l'eau plus un additif dissout dans l'eau. Pour éliminer le CO₂, l'additif est par exemple de la soude (NaOH), de la potasse (KOH), de la chaux vive ou éteinte (CaO, Ca(OH)2).

Pour éliminer l'éthylène, l'additif contient un oxydant fort, par exemple du permanganate (MnO4⁻) ou du bichromate (Cr₂O₇²⁻). Typiquement, l'additif contient du permanganate de sodium ou du permanganate de potassium ou du bichromate de potassium.

Le liquide peut contenir seulement un additif destiné à éliminer le CO₂, ou seulement un additif destiné à éliminer l'éthylène, ou contenir un additif pour éliminer le CO₂ et un additif pour éliminer l'éthylène.

La concentration des différents additifs est choisie en fonction de la quantité de CO₂ et de la quantité d'éthylène à éliminer.

Par exemple, le liquide est une solution aqueuse de permanganate de sodium ou de permanganate de potassium, saturée en permanganate.

Comme précisé plus haut, le CO₂ et l'éthylène sont dégagés par les produits végétaux lors de leur maturation.

Ainsi, dans la variante du second mode de réalisation de l'invention, le dispositif et le procédé permettent d'éliminer les poussières, les arômes, le CO₂ et/ou l'éthylène de l'atmosphère. Ils permettent également de réhumidifier l'atmosphère et de réenrichir l'atmosphère en eau en O₂.

Dans ce cas, le capteur 55 est de préférence configuré pour analyser la ou les concentrations en CO₂ et/ou en éthylène dans l'atmosphère du stockage. Le dispositif électronique 51 est configuré pour régler le débit du flux de liquide en fonction de la ou les concentrations mesurées.

Le procédé de traitement de l'atmosphère du stockage selon le second mode de réalisation comprend ainsi une étape de mise en contact de l'atmosphère avec un flux de liquide par circulation dans un garnissage, le liquide étant de l'eau contenant éventuellement un additif.

Typiquement, le procédé comprend une étape de mesure de la ou les concentrations de O₂ et/ou de CO₂ et/ou en éthylène dans l'atmosphère, et/ou de mesure de la température de l'atmosphère, et/ou de mesure de l'humidité dans l'atmosphère du stockage, le débit du flux de liquide et/ou le débit de l'atmosphère à l'étape de mise en contact étant ajustés en fonction de la ou des mesures.

De préférence, le liquide injecté est collecté sous le garnissage, puis évacué vers les égouts. Il n'est pas recyclé.

Le procédé est prévu pour être mis en œuvre par l'ensemble de traitement 1 décrit ci-dessus. Inversement, l'ensemble de traitement 1 décrit ci-dessus est particulièrement adapté pour la mise en œuvre du procédé.

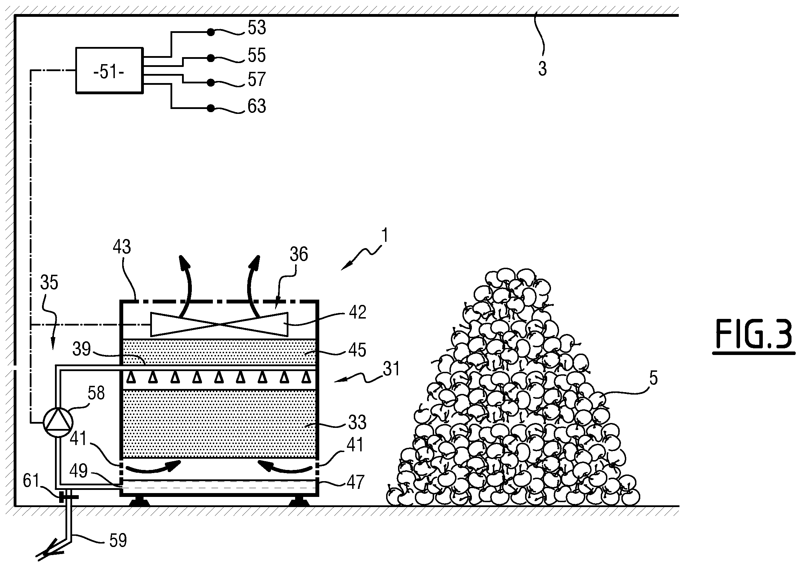

Une autre variante du second mode de réalisation va maintenant être décrite, en référence à la figure 3. Seuls les points par lesquels cette variante diffère de celle de la figure 2 seront détaillés ci-dessous. Les éléments identiques et assurant la même fonction seront désignés par la même référence.

Dans cette autre variante, le dispositif et le procédé de l'invention visent principalement à éliminer l'éthylène de l'atmosphère du stockage.

Le dispositif d'injection 35, outre le ou les organes d'aspersion 39, comporte un organe de transfert 58 tel qu'une pompe, aspirant le liquide dans le bac 47 et refoulant celui-ci dans le ou les organes d'aspersion 39.

Pour éliminer l'éthylène, le liquide comporte un additif contenant un oxydant fort tel que du permanganate (MnO4⁻) ou du bichromate (Cr₂O₇²⁻). Typiquement, l'additif contient du permanganate de sodium ou du permanganate de potassium ou du bichromate de potassium.

En variante, le liquide comporte un ou plusieurs additifs supplémentaires. Par exemple, le liquide comprend un sel métallique permettant d'augmenter la vitesse de réaction de l'oxydant avec l'éthylène (catalyseur). Ce catalyseur est typiquement du sulfate de fer quand l'oxydant est le permanganate de potassium. Selon un autre exemple, l'additif supplémentaire est prévu pour éliminer le CO₂.

Par exemple, le liquide est une solution aqueuse de permanganate de sodium ou de permanganate de potassium, sursaturée en permanganate. En d'autres termes, pour un bac 47 contenant un volume d'eau V donné, on mélange au départ à l'eau une masse de permanganate M supérieure à Cₛₐₜ x V, où Cₛₐₜ est la concentration de saturation du permanganate dans l'eau. Une partie du permanganate n'est pas dissoute initialement et reste sous forme de bioxyde MnO₂ solide dans le bac 47. Puis, au fur et à mesure que le dispositif fonctionne, le permanganate dissout dans l'eau est consommé par réaction avec l'éthylène contenu dans l'atmosphère du stockage. Une fraction du bioxyde MnO₂ solide passe en solution, de telle sorte que la concentration de permanganate reste la concentration de saturation. Par exemple, la masse initiale de permanganate est choisie pour que le dispositif puisse être utilisé pendant toute la période de stockage sans avoir à changer le liquide. A la fin de la période de stockage, le liquide est envoyé à un centre de récupération des sels de manganèse.

Le bac 47 contient typiquement entre 1 m³ et 3 m³ de liquide, en fonction de la taille de la chambre de stockage et de la capacité de traitement du dispositif/procédé. Pour 2 m³ de liquide, on mélange initialement par exemple 150 kg de permanganate. Environ 60 kg de permanganate seront dissouts, les 90 kg restant constituant une réserve pour maintenir la concentration de permanganate à saturation pendant la période de stockage.

Une vidange 59 permet de décharger le liquide contenu dans le bac 47 vers les égouts, typiquement quand la concentration en permanganate a diminuée de manière excessive. Le liquide usé contenant les produits indésirables est ainsi déchargé périodiquement du dispositif de mise en contact 31 vers les égouts. La vidange 59 est normalement fermée par une vanne 61. Des moyens non représentés sont prévus pour remplir le bac 47 avec une charge de liquide frais.

Typiquement, l'ensemble de traitement est mis en fonctionnement périodiquement, par exemple une fois par jour, de manière à assainir l'atmosphère du stockage.

Le débit de liquide est typiquement compris entre 5 et 50 m³/heure, le débit de l'atmosphère à travers le garnissage étant compris entre 1 et 10 volume/heure, c'est-à-dire entre 2000 et 20 000 m³/heure pour un volume à traiter de 2000 m³.

La vitesse de l'air dans le garnissage doit permettre d'assurer un contact intime avec le liquide, de manière à assurer l'oxydation complète de l'éthylène. Les débits de liquide et d'air sont donc choisis en fonction de la section du garnissage, de manière à réaliser ce contact et à assurer un débit d'air très élevé, ce qui permet de maintenir la concentration d'éthylène dans la chambre à un niveau extrêmement bas. En revanche, la vitesse de l'air reste inférieure à 3,5 m/sec pour éviter l'entrainement de gouttes de liquide.

Par exemple, pour des chambres jusqu'à 2000 m³ , le débit d'air est de l'ordre de 10.000 m³/h et le garnissage présente une section de 0,9 mx1,2 m, soit une vitesse d'air de 2.57 m/sec environ.

Pour des chambres entre 2000 m³ et 4000 m³, la section du garnissage est par exemple 1,2 mx1,2 m et le débit d'air de 16.000 m³/ h , soit 3,09 m/ sec.

Avantageusement, l'ensemble 1 comporte un capteur 63, configuré pour analyser la concentration en éthylène dans l'atmosphère du stockage, typiquement un analyseur de gaz. Le dispositif électronique 51 est configuré pour régler le débit du flux de liquide en fonction de la concentration mesurée.

Il est à noter que la présente variante de réalisation, comme décrit plus haut, permet aussi d'éliminer les poussières, les arômes, et éventuellement le CO₂ de l'atmosphère. Elle permet en outre de réhumidifier l'atmosphère et contribue à contrôler la température de l'atmosphère.

De préférence, l'ensemble 1 comporte alors un capteur 53 de mesure de la température à l'intérieur du stockage 3, et/ou un capteur 55 configuré pour analyser la ou les concentrations en O₂ et/ou en éthylène dans l'atmosphère du stockage, et/ou un hygromètre 57, agencé pour mesurer l'humidité de l'atmosphère dans la chambre 3. Le dispositif électronique 51 est configuré pour régler le débit du flux de liquide et/ou le débit de l'atmosphère en fonction des mesures renvoyées par les capteurs 53, 55, 57 et 63.

Le procédé de traitement de l'atmosphère du stockage selon l'autre variante du second mode de réalisation comprend ainsi une étape de mise en contact de l'atmosphère avec un flux de liquide par circulation dans un garnissage, le liquide contenant au moins un additif tel que décrit ci-dessus.

Typiquement, le procédé comprend une étape de mesure de la ou les concentrations de O₂ et/ou de CO₂ et/ou d'éthylène dans l'atmosphère, et/ou de mesure de la température de l'atmosphère, et/ou de mesure de l'humidité dans l'atmosphère du stockage, le débit du flux de liquide et/ou le débit de l'atmosphère à l'étape de mise en contact étant ajustés en fonction de la ou des mesures.

De préférence, le liquide injecté est collecté sous le garnissage, puis recyclé à l'étape de mise en contact.

Le procédé est prévu pour être mis en œuvre par l'ensemble de traitement 1 décrit ci-dessus. Inversement, l'ensemble de traitement 1 décrit ci-dessus est particulièrement adapté pour la mise en œuvre du procédé.

Il est à noter que le liquide pourrait ne pas être recyclé, mais plutôt être vidangé directement du bac 47, comme sur la figure 2, après un seul passage dans le dispositif de mise en contact.

L'invention permet de maintenir le taux d'éthylène dans l'atmosphère du stockage à des valeurs extrêmement basses, inférieures à 1 ppm.

Les méthodes utilisées actuellement dans ce but présentent des défauts importants.

Une méthode existante consiste à bloquer les récepteurs de l'éthylène dans les végétaux stockés. Ces végétaux cessent alors de produire de l'éthylène. On utilise typiquement le 1MCP (méthylcyclopropène) dans ce but. L'inconvénient de cette méthode est que l'évolution des végétaux est définitivement bloquée, celui-ci ne pouvant plus mûrir normalement.

D'autres méthodes consistent à détruire les molécules d'éthylène dans l'atmosphère, soit par oxydation chimique soit par combustion catalytique. Ces méthodes ne permettent pas de traiter des débits d'air suffisants pour abaisser la concentration d'éthylène à moins de 1 ppm dans des stockages de vastes volumes, d'au moins 1000 m³.

Un tel taux contribue à ralentir de manière importante la maturation des végétaux stockés. Les végétaux sont mieux conservés et une plus grande proportion de ceux-ci sont donc aptes à la commercialisation au moment de l'ouverture du stockage. Les pertes par pourritures sont réduites. Le développement des maladies physiologiques entraînées par l'évolution des végétaux stockés est ralenti ou empêché. L'absence de produits sénescents favorise la conservation. Par ailleurs, si l'absorption de l'éthylène est interrompue, les végétaux reprennent le cours normal de leur maturation.

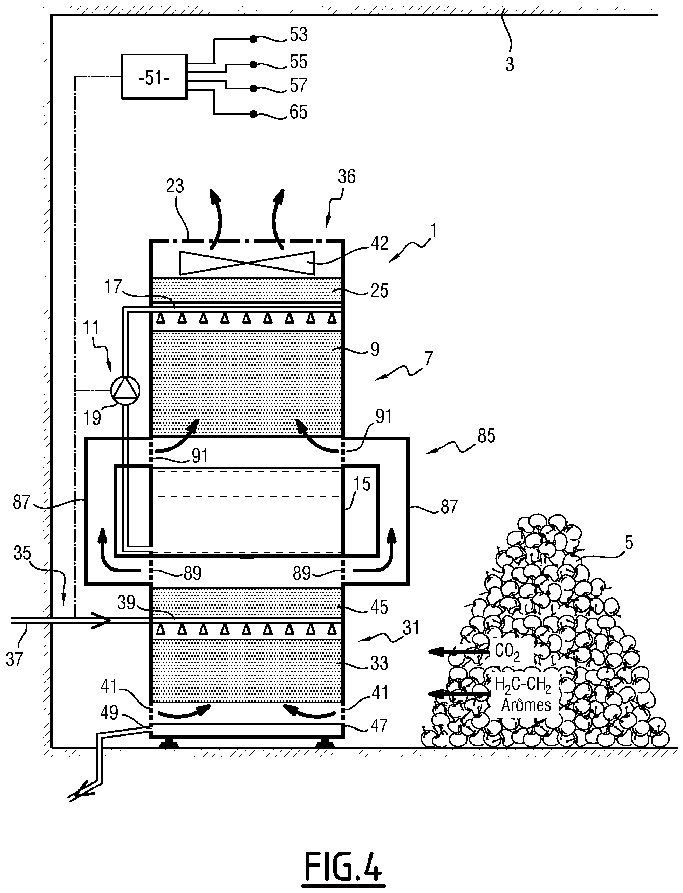

Un troisième mode de réalisation de l'invention va maintenant être décrit, en référence à la figure 4. Les éléments identiques ou assurant les mêmes fonctions que dans les deux premiers modes de réalisation seront désignés par les mêmes références.

Dans le troisième mode de réalisation l'ensemble 1 comprend le dispositif de mise en contact 31 et le dispositif d'injection 35 du second mode de réalisation.

Il comporte également un autre dispositif de mise en contact et un autre dispositif d'injection d'un liquide, conformes à ceux du premier mode de réalisation, désignés ici par les références 7 et 11 comme dans le premier mode de réalisation.

L'ensemble 1 comporte encore un dispositif 85 configuré pour faire circuler l'atmosphère sortant du dispositif de mise en contact 31 dans l'autre dispositif de mise en contact 11.

L'atmosphère sortant du dispositif 31 est ainsi mise en contact avec l'autre flux de liquide par circulation dans le garnissage 9.

Le liquide injecté dans le garnissage 33 est de l'eau, avec éventuellement un ou plusieurs additifs tels que décrits ci-dessus pour piéger le CO₂ et/ou l'éthylène. L'eau est à une température inférieure à 50°C, typiquement à température ambiante.

L'autre liquide comprend au moins un produit biocide et/ou phytoprotecteur volatile, de température d'ébullition comprise entre 60 et 280°C, cet autre liquide étant évaporé dans l'autre dispositif de mise en contact 7 à une température inférieure à 50°C.

Comme visible sur la figure 4, typiquement, le dispositif de mise en contact 31 et l'autre dispositif de mise en contact 7 sont empilés l'un au-dessus de l'autre. Ils forment une tour verticale, le dispositif de mise en contact 31 formant la partie inférieure de la tour, et l'autre dispositif de mise en contact 7 formant la partie supérieure de la tour. En variante, ils sont disposés dans deux tours distinctes.

L'ensemble 1 comporte encore un organe de circulation 42 situé au sommet de l'empilement, au-dessus des garnissages de l'autre dispositif de mise en contact 7. Il permet de faire circuler l'atmosphère à la fois à travers le dispositif de mise en contact 31 et à travers l'autre dispositif de mise en contact 7.

La réserve 15 de l'autre dispositif de mise en contact est située au-dessus du séparateur de gouttes 45 du dispositif de mise en contact.

Le dispositif 85 configuré pour faire circuler l'atmosphère sortant du dispositif de mise en contact 31 jusqu'à l'autre dispositif de mise en contact 7, comprend un ou plusieurs passages 87 raccordant des sorties 89 du dispositif de mise en contact à des entrées 91 de l'autre dispositif de mise en contact. Les sorties 89 sont situées verticalement entre le garnissage 33 et la réserve 15, et plus précisément verticalement entre le séparateur de gouttes 45 et la réserve 15. Les entrées 91 sont situées sous le garnissage 9, et plus précisément entre la réserve 15 et le garnissage 9.

Les entrées et les sorties 89, 91 sont ménagées dans les parois latérales de la tour.

L'atmosphère pénètre dans le dispositif de mise en contact 31 par les entrées 41 et circule vers le haut à travers le garnissage 33. Le liquide arrivant par le dispositif d'injection 35 s'écoule à travers le garnissage 33 à contre-courant du sens de circulation de l'atmosphère. Les poussières, les arômes, éventuellement le CO₂ et/ou l'éthylène, sont transférés au moins partiellement de l'atmosphère vers le liquide lors de la mise en contact au niveau des garnissages 33. L'atmosphère passe ensuite à travers le séparateur de gouttes 45, puis traverse les entrées 89 et circule dans les passages 87 jusqu'aux entrées 91.

L'atmosphère circule alors vers le haut à travers le garnissage 9 de l'autre dispositif de mise en contact. L'autre liquide, contenant le produit biocide et/ou phytoprotecteur, est injecté par le dispositif 11 au-dessus du garnissage 9. Il circule vers le bas, et est mis en contact avec l'atmosphère purifiée dans le garnissage 9. Au moins une partie du produit est transférée depuis le liquide dans l'atmosphère. L'atmosphère chargée en produits biocides et/ou phytoprotecteurs, en quittant le garnissage 79, circule vers le haut à travers le séparateur de gouttes puis à travers la sortie 23 et est rejetée dans le stockage.

Le dispositif électronique 51 pilote l'ensemble 1 comme décrit précédemment, en référence aux figures 1 et 2. A cet effet, l'ensemble 1 comporte avantageusement le capteur de température 53, le capteur 55 configuré pour analyser la ou les concentrations en O₂ et/ou en CO₂ et/ou éthylène dans l'atmosphère du stockage, l'hygromètre 57, et un capteur 65 de mesure de la concentration du ou de chaque produit dans l'atmosphère.

Le procédé de traitement de l'atmosphère du stockage selon le troisième mode de réalisation comprend ainsi :
- au moins une étape de mise en contact de l'atmosphère avec un flux de liquide par circulation dans un garnissage, le liquide étant de l'eau contenant éventuellement un additif ;
- après l'étape de mise en contact, une autre étape de mise en contact de l'atmosphère avec au moins un flux d'un autre liquide par circulation dans un autre garnissage, l'autre liquide comprenant au moins un produit biocide et/ou phytoproducteur volatile, de température d'ébullition comprise entre 60 et 280°C, l'autre liquide étant évaporé à l'autre étape de mise en contact à une température inférieure à 50°C.

Quand le liquide est de l'eau, l'étape de mise en contact vise à éliminer seulement les poussières et les arômes de l'atmosphère. Quand le liquide contient un additif tel qu'un sel alcalin, l'étape de mise en contact permet d'éliminer également le CO₂ de l'atmosphère. Quand le liquide contient un additif tel que du permanganate, l'étape de mise en contact permet d'éliminer l'éthylène de l'atmosphère.

L'autre étape de mise en contact permet d'évaporer le ou chaque produit biocide et/ou phytoprotecteur.

Typiquement, le procédé comprend une étape de mesure de la ou des concentrations de O₂ et/ou de CO₂ et/ou d'éthylènes et/ou du ou de chaque produit biocide et/ou phytoproducteur dans l'atmosphère, et/ou de mesure de la température de l'atmosphère, et/ou de mesure de l'humidité dans l'atmosphère du stockage, le débit du flux de liquide et/ou le débit de l'atmosphère à l'étape de mise en contact étant ajustés en fonction de la ou des mesures.

De préférence, le liquide injecté est collecté sous le garnissage, puis évacué vers les égouts. Il n'est pas recyclé. En revanche, l'autre liquide injecté est typiquement collecté sous l'autre garnissage, dans une réserve, et recyclé à l'autre étape de mise en contact.

Le procédé est prévu pour être mis en œuvre par l'ensemble de traitement 1 décrit ci-dessus. Inversement, l'ensemble de traitement 1 décrit ci-dessus est particulièrement adapté pour la mise en œuvre du procédé.

L'ensemble 1 présente dans le troisième mode de réalisation de multiples avantages.

En effet, le dispositif de mise en contact 31 permet d'éliminer au moins les arômes et la poussière, et typiquement également le CO₂ et/ou l'éthylène. L'autre dispositif de mise en contact 11 permet d'évaporer le produit de traitement.

Dans l'état de la technique, le CO₂ et/ou l'éthylène dégagés par les produits végétaux sont éliminés en ouvrant les portes du stockage, de manière à renouveler au moins partiellement l'atmosphère à l'intérieur de celui-ci. Ceci entraîne à l'extérieur du stockage le produit de traitement qui se trouve dispersé dans l'atmosphère. Une fois les portes refermées, il est nécessaire de vaporiser une quantité importante de produits biocides et/ou phytoprotecteurs, de manière à restaurer la concentration de produits souhaités dans l'atmosphère.

Du fait que l'ensemble 1 permet d'éliminer les produits gênants s'accumulant dans l'atmosphère, il n'est plus nécessaire d'ouvrir les portes, périodiquement, de telle sorte que la consommation de produits biocides et/ou phytoprotecteurs est réduite.

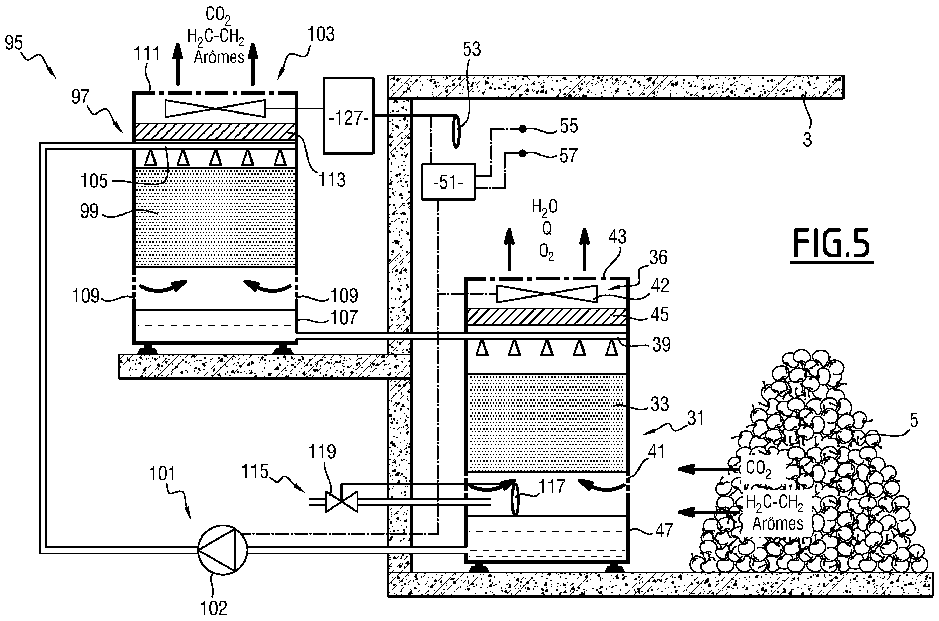

Un quatrième mode de réalisation de l'invention va maintenant être décrit, en référence à la figure 5. Seuls les points par lesquels ce quatrième mode de réalisation diffère du second mode de réalisation seront détaillés ci-dessous.

Les éléments identiques ou assurant les mêmes fonctions seront désignés par les mêmes références dans les deux modes de réalisation.

Dans le quatrième mode de réalisation, l'ensemble 1 comprend, outre le dispositif de mise en contact 31 et le dispositif de circulation 36, un dispositif de régénération 95 du flux de liquide.

Le dispositif de régénération 95 comporte :
- un dispositif extérieur de mise en contact 97, comportant un garnissage extérieur 99 ;
- un dispositif 101 d'injection du flux de liquide provenant du dispositif de mise en contact 31 dans le dispositif extérieur 97 de mise en contact ;
- un dispositif 103 de circulation d'un flux d'air extérieur au stockage dans le dispositif extérieur 97 de mise en contact.

Le dispositif extérieur 97 de mise en contact est conformé pour que le flux de liquide soit mis en contact avec un flux d'air extérieur au stockage par circulation dans le garnissage extérieur 99 dans le dispositif de mise en contact 97.

Le dispositif extérieur de mise en contact 97 est du même type que le dispositif de mise en contact 31. Il s'agit typiquement d'une tour à garnissage.

Le dispositif 101 d'injection du flux de liquide aspire le liquide collecté dans le bac 47 placé sous le garnissage 33. Il comporte typiquement un organe de transfert 102 tel qu'une pompe qui refoule le liquide jusqu'à des organes d'aspersion 105 tels que des rampes, placées au-dessus du garnissage 99. Le dispositif extérieur de mise en contact 97 présente sous le garnissage extérieur 99 un bac 107 collectant le liquide s'écoulant à travers le garnissage 99. Ce liquide est renvoyé vers les organes d'aspersion 39 du dispositif de mise en contact 31, par tous moyens adaptés, par exemple gravitairement ou à l'aide d'une pompe non représentée.

Le dispositif de circulation 103 comprend typiquement un ventilateur ou une soufflante, qui crée un courant d'air extérieur ascendant à travers le garnissage 99. L'air extérieur pénètre dans le dispositif extérieur de mise en contact 97 par des entrées 109 situées sous le garnissage 99. Il sort par une sortie 111 située au sommet du dispositif extérieur de mise en contact 97.

Ce dispositif 97 comporte encore un séparateur de gouttes 113 situé au-dessus des organes d'aspersion 105.

Le dispositif de mise en contact 31 comporte avantageusement une alimentation d'appoint en liquide 115. Cette alimentation 115 est raccordée à un réseau de fourniture de liquide. Le dispositif de mise en contact 31 comprend un capteur 117 du niveau de liquide dans le bac 47, qui pilote une vanne 119 placée sur l'alimentation d'appoint 115. Le capteur de niveau 117 est configuré pour que la vanne 119 soit ouverte quand le niveau de liquide dans le bac 47 est inférieur à un niveau prédéterminé, et fermée quand le niveau est supérieur à un niveau prédéterminé.

De préférence, l'ensemble 1 comporte un capteur 53 de mesure de la température à l'intérieur du stockage 3. Le débit d'air extérieur à l'étape de régénération est réglé au moins en fonction de la température mesurée.

Ce débit est réglé soit par un thermostat 127 pilotant directement l'organe de circulation 103, soit par le dispositif de pilotage 51 qui est programmé pour gérer l'organe de circulation 103.

Avantageusement, l'ensemble 1 comporte un capteur 55, configuré pour analyser la ou les concentrations en CO₂ et/ou en O₂ et/ou en éthylène dans l'atmosphère du stockage. Le dispositif de pilotage 51 est programmé pour régler le débit du flux de liquide en fonction de la ou des concentrations mesurées.

Le dispositif de pilotage 51 pour cela pilote l'organe de transfert 102.

Par exemple, l'ensemble 1 comporte un hygromètre 57, agencé pour mesurer l'humidité de l'atmosphère dans la chambre 3. Le dispositif de pilotage 51 est programmé pour régler le débit de l'atmosphère à l'étape de mise en contact en fonction de l'humidité mesurée par l'hygromètre 57.

En variante, le débit de l'atmosphère à l'étape de mise en contact est réglé en fonction de la ou des concentrations en CO₂ et/ou en éthylène mesurées par le capteur 55 dans l'atmosphère du stockage 3.

Chaque dispositif de mise en contact présente une section horizontale comprise entre 0,5 m² et 20 m². Leur hauteur est comprise entre 2 mètres et 6 mètres. Le débit de liquide est typiquement compris entre 20 m³/heure et 1000 m³/heure, le débit de l'atmosphère dans le dispositif de mise en contact 31 étant compris entre 2000 et 100 000 m³/heure.

Le procédé de traitement selon le quatrième mode de réalisation comprend :
- une étape de mise en contact de l'atmosphère du stockage 3 avec le flux de liquide par circulation dans un garnissage 33 ;
- une étape de collecte du flux de liquide issu de l'étape de mise en contact ;
- une étape de régénération du flux de liquide issu de l'étape de mise en contact, au cours de laquelle le flux de liquide est mis en contact avec un flux d'air extérieur au stockage par circulation dans un garnissage extérieur 99, le flux de liquide étant ensuite réutilisé à l'étape de mise en contact.

Le flux de liquide est typiquement un flux d'eau, sans additif. En effet, un tel système permet de travailler en circuit fermé avec une grande quantité d'eau, ce qui fait que les additifs prévus pour éliminer le CO₂ et/ou l'éthylène dans d'autres modes de réalisation, ne sont pas nécessaires ici.

Comme décrit plus haut, le procédé comprend avantageusement une étape de mesure de la ou des concentrations en CO₂ et/ou en O₂ et/ou en éthylène dans l'atmosphère du stockage, le débit du flux de liquide étant réglé en fonction de la ou des concentrations mesurées. De même, le procédé comprend avantageusement une étape de mesure de la température et/ou de l'humidité dans l'atmosphère du stockage, le débit de l'atmosphère à l'étape de mise en contact étant réglé en fonction de ladite température et/ou humidité mesurée.

De même, le procédé comprend de préférence une étape de mesure de la température dans l'atmosphère du stockage, le débit du flux d'air extérieur à l'étape de régénération étant réglé au moins en fonction de ladite température mesurée.

Dans ce mode de réalisation, le CO₂, l'éthylène et les arômes produits par la respiration des produits végétaux stockés dans le stockage sont absorbés par le flux de liquide à l'étape de mise en contact. A la même étape, le liquide arrivant de l'extérieur du stockage est partiellement vaporisé et contribue à maintenir une humidité suffisante dans l'atmosphère du stockage. De plus, le liquide arrive de l'extérieur avec une concentration en oxygène dissous significative, cet oxygène étant au moins partiellement relargué dans l'atmosphère du stockage au cours de l'étape de mise en contact du liquide avec l'atmosphère.

Dans certaines circonstances, en fonction de la température et de l'humidité à l'intérieur et à l'extérieur du stockage, l'étape de mise en contact produira un effet de refroidissement par évaporation d'eau à l'intérieur du stockage. Ceci contribue à limiter la charge du système de conditionnement d'air du stockage 3.

Le liquide collecté dans le bac 47 du dispositif de mise en contact est dirigé vers le dispositif extérieur de mise en contact 95. Dans ce dispositif, les arômes, l'éthylène et le CO₂ sont relargués dans l'air extérieur. Le liquide est réoxygéné.

Le liquide collecté dans le bac 107 alimente le dispositif de mise en contact 31.

Le procédé est prévu pour être mis en œuvre par l'ensemble de traitement 1 décrit ci-dessus. Inversement, l'ensemble de traitement 1 décrit ci-dessus est particulièrement adapté pour la mise en œuvre du procédé.

On conçoit donc que l'ensemble et le procédé selon le quatrième mode de réalisation permet de réguler, ou au moins contribue à réguler, un grand nombre de paramètres importants pour la conservation et le processus de maturation des produits végétaux dans le stockage. Ces paramètres sont la concentration en CO₂, et/ou en éthylène et/ou en oxygène dans l'atmosphère du stockage, l'élimination des arômes, l'humidité dans l'atmosphère du stockage et la température à l'intérieur du stockage.

Comme souligné précédemment, il permet d'éviter l'ouverture périodique des portes du stockage pour chasser le CO₂ accumulé, en le remplaçant par de l'air frais extérieur.

Cet ensemble et ce procédé s'appliquent par exemple au stockage de pommes de terre.

Dans ce cas, l'atmosphère du stockage est de l'air. Le débit de l'atmosphère dans le dispositif de mise en contact 31 est réglé en fonction de l'humidité mesurée par l'hygromètre dans l'atmosphère de la chambre. Le débit d'air extérieur est contrôlé en fonction de la température mesurée dans l'atmosphère de la chambre.

Le dispositif et le procédé sont également applicables au stockage de pommes sous atmosphère contrôlée. L'atmosphère de la chambre est maintenue avec un taux d'oxygène de l'ordre de 2%. Le taux de CO₂ est maintenu entre 2 et 5%, c'est-à-dire à une valeur élevée. La température est maintenue à une valeur basse, de l'ordre de 0,5°C.

Le débit de l'atmosphère à travers le dispositif de mise en contact est réglé en fonction de la concentration en CO₂ mesurée par le capteur 55 dans l'atmosphère de la chambre. Le débit de liquide est réglé en fonction de la concentration de CO₂ et/ou de O₂ mesurée par le capteur 55 dans l'atmosphère de la chambre. Le débit d'air extérieur dans le dispositif extérieur de mise en contact 95 est réglé en fonction de la température dans l'atmosphère du stockage.

Il est à noter que, en variante, l'ensemble 1 selon le quatrième mode de réalisation de l'invention, peut avantageusement comporter en lieu et place du dispositif de mise en contact 31 du type illustré sur la figure 2, un sous-ensemble du type illustré sur la figure 4, avec un dispositif de mise en contact de l'atmosphère avec un flux d'eau provenant du dispositif extérieur 95, et un autre dispositif de mise en contact avec un liquide comprenant un produit biocide et/ou phytoprotecteur.

Il est important de noter également que, dans tous les modes de réalisation, le système s'autorégule. Le produit biocide et/ou phytoprotecteur étant vaporisé à température ambiante, inférieure à 50°C, il s'évapore jusqu'à atteindre la saturation de l'atmosphère sans risque de sursaturation (ce qui est le cas quand on chauffe). Cela permet d'éviter que le liquide recondense après injection. Ceci est vrai également pour l'évaporation d'eau permettant d'humidifier l'atmosphère.

Ainsi, en plus ou à la place de la régulation par mesure de la concentration du produit ou de l'humidité dans l'atmosphère, il est envisagé de laisser le système s'autoréguler. Le système fonctionne par exemple en permanence. L'eau ou le produit à température ambiante s'évapore jusqu'à atteindre la saturation. Une fois la saturation atteinte, il n'y a plus d'évaporation.

## Revendications

1. Procédé de traitement de l'atmosphère d'un stockage (3) de produits végétaux (5) ayant un volume supérieur à 1000 m³, le procédé comprenant au moins une étape de mise en contact de l'atmosphère avec un flux de liquide par circulation dans un garnissage, le liquide comprend au moins un produit biocide et/ou phytoprotecteur volatile, de température d'ébullition comprise entre 60 et 280°C, le liquide étant évaporé à l'étape de mise en contact à une température inférieure à 50°C ; l'au moins un produit phytoprotecteur étant choisi dans la liste suivante : huile essentielle, terpènes, alcool de C3 à C9 saturé ou insaturé, le glutaraldéhyde, l'hexanal, le diméthylnaphtalène et le 3-décène-2-one ; l'au moins un produit biocide étant choisi dans la liste suivante : l'huile de girofle, l'huile de thym, le geraniol, l'alcool ethylique, le glutaraldéhyde.

2. Procédé selon la revendication 1, **caractérisé en ce que** le procédé comprend :
- une étape de collecte du flux de liquide issu de l'étape de mise en contact ;
- une étape de régénération du flux de liquide issu de l'étape de mise en contact, au cours de laquelle ledit flux de liquide est mis en contact avec un flux d'air extérieur au stockage par circulation dans un garnissage extérieur, le flux de liquide étant ensuite réutilisé à l'étape de mise en contact.

3. Procédé selon la revendication 2, **caractérisé en ce que** le procédé comprend une étape de mesure de la concentration en CO₂ et/ou O₂ dans l'atmosphère du stockage, un débit du flux de liquide étant réglé en fonction de ladite concentration mesurée.

4. Procédé selon la revendication 2 ou 3, **caractérisé en ce que** le procédé comprend une étape de mesure de la température et/ou de l'humidité dans l'atmosphère du stockage, un débit de l'atmosphère à l'étape de mise en contact étant réglé en fonction de ladite température et/ou humidité mesurée.

5. Procédé selon l'une quelconque des revendications 2 à 4, **caractérisé en ce que** le procédé comprend une étape de mesure de la température dans l'atmosphère du stockage, un débit du flux d'air extérieur à l'étape de régénération étant réglé au moins en fonction de ladite température mesurée.

6. Ensemble de traitement de l'atmosphère d'un stockage (3) de produits végétaux (5) ayant un volume supérieur à 1000 m³, l'ensemble (1) comprenant au moins :
- un dispositif (7, 31) de mise en contact, comportant un garnissage (9, 33);
- un dispositif (11, 35) d'injection d'un flux de liquide dans le dispositif de mise en contact (7, 31) ;
- un dispositif (13, 36) de circulation de l'atmosphère du stockage (3) dans le dispositif de mise en contact (7, 31) ;
le dispositif de mise en contact (7, 31) étant conformé pour que l'atmosphère soit mise en contact avec le flux de liquide par circulation dans le garnissage (9, 33),
le dispositif de mise en contact (7) comporte une réserve (15) et une dose de liquide stockée dans la réserve, le liquide comprenant au moins un produit biocide et/ou phytoprotecteur volatile, de température d'ébullition comprise entre 60 et 280°C, le liquide étant évaporé dans le dispositif de mise en contact (7) à une température inférieure à 50°C,
l'au moins un produit phytoprotecteur étant choisi dans la liste suivante : huile essentielle, terpènes, alcool de C3 à C9 saturé ou insaturé, le glutaraldéhyde, l'hexanal, le diméthylnaphtalène et le 3-décène-2-one ; l'au moins un produit biocide étant choisi dans la liste suivante : l'huile de girofle, l'huile de thym, le geraniol, l'alcool ethylique, le glutaraldéhyde.

7. Ensemble selon la revendication 6, **caractérisé en ce que** l'ensemble comprend un dispositif (95) de régénération du flux de liquide, avec :
- un dispositif extérieur (97) de mise en contact, comportant un garnissage extérieur (99) ;
- un dispositif (101) d'injection du flux de liquide provenant du dispositif de mise en contact (31) dans le dispositif extérieur de mise en contact (95) ;
- un dispositif (103) de circulation d'un flux d'air extérieur au stockage (3) dans le dispositif extérieur de mise en contact (95);
le dispositif extérieur de mise en contact (95) étant conformé pour que le flux de liquide soit mis en contact avec un flux d'air extérieur au stockage (3) par circulation dans le garnissage extérieur (99), puis recyclé dans le dispositif de mise en contact (31).

## Patentansprüche

1. Verfahren zur Behandlung der Lagerungsatmosphäre (3) von Pflanzenerzeugnissen (5), das ein Volumen von mehr als 1000 m³ aufweist, wobei das Verfahren mindestens einen Schritt eines Inkontaktbringens der Atmosphäre mit einem Flüssigkeitsstrom durch Zirkulation in einer Packung umfasst, die Flüssigkeit mindestens ein flüchtiges Biozid- und/oder Pflanzenschutzmittel mit einer Siedetemperatur zwischen 60 und 280 °C umfasst, wobei die Flüssigkeit bei dem Schritt eines Inkontaktbringens bei einer Temperatur von unter 50 °C verdampft wird; wobei das mindestens eine Pflanzenschutzmittel ausgewählt ist aus der folgenden Liste: ätherisches Öl, Terpene, gesättigter oder ungesättigter C3-C9-Alkohol, Glutaraldehyd, Hexanal, Dimethylnaphthalin und 3-Decen-2-on; wobei das mindestens eine Biozidmittel ausgewählt ist aus der folgenden Liste: Nelkenöl, Thymianöl, Geraniol, Ethylalkohol, Glutaraldehyd.

2. Verfahren nach Anspruch 1, **dadurch gekennzeichnet, dass** das Verfahren Folgendes umfasst:
- einen Schritt eines Sammelns des Flüssigkeitsstroms aus dem Schritt des Inkontaktbringens;
- einen Schritt eines Regenerierens des Flüssigkeitsstroms aus dem Schritt des Inkontaktbringens, bei dem der Flüssigkeitsstrom durch Zirkulation in einer äußeren Packung mit einem Luftstrom außerhalb des Lagers in Kontakt gebracht wird, wobei der Flüssigkeitsstrom anschließend wieder in dem Schritt des Inkontaktbringens verwendet wird.

3. Verfahren nach Anspruch 2, **dadurch gekennzeichnet, dass** das Verfahren einen Schritt des Messens der CO₂- und/oder O₂-Konzentration in der Atmosphäre des Speichers umfasst, wobei eine Durchflussrate des Flüssigkeitsstroms abhängig von der gemessenen Konzentration eingestellt wird.

4. Verfahren nach Anspruch 2 oder 3, **dadurch gekennzeichnet, dass** das Verfahren einen Schritt eines Messens der Temperatur und/oder der Feuchtigkeit in der Atmosphäre des Lagers umfasst, eine Durchflussrate der Atmosphäre beim Schritt des Inkontaktbringens abhängig von der gemessenen Temperatur und/oder Feuchtigkeit eingestellt wird.

5. Verfahren nach einem der Ansprüche 2 bis 4, **dadurch gekennzeichnet, dass** das Verfahren einen Schritt zum Messen der Temperatur in der Atmosphäre des Lagers umfasst, wobei eine Durchflussrate des Außenluftstroms in der Regenerationsstufe zumindest abhängig von der gemessenen Temperatur eingestellt wird.

6. Anordnung zur Behandlung der Lagerungsatmosphäre (3) von Pflanzenerzeugnissen (5), das ein Volumen von mehr als 1000 m³ aufweist, wobei die Anordnung (1) mindestens Folgendes umfasst:
- eine Vorrichtung (7, 31) zum Inkontaktbringen, umfassend eine Packung (9, 33);
- eine Vorrichtung (11, 35) zum Einspritzen eines Flüssigkeitsstroms in die Vorrichtung zum Inkontaktbringen (7, 31);
- eine Vorrichtung (13, 36) zum Zirkulieren der Atmosphäre des Lagers (3) in der Vorrichtung zum Inkontaktbringen (7, 31);
wobei die Vorrichtung zum Inkontaktbringen (7, 31) ausgebildet ist, damit die Atmosphäre durch Zirkulation in der Packung (9, 33) mit dem Flüssigkeitsstrom in Kontakt gebracht wird,
die Vorrichtung zum Inkontaktbringen (7) einen Vorrat (15) und eine in dem Vorrat gespeicherte Flüssigkeitsdosis umfasst, die Flüssigkeit mindestens ein flüchtiges Biozid- und/oder Pflanzenschutzmittel mit einer Siedetemperatur zwischen 60 und 280 °C umfasst, wobei die Flüssigkeit in der Vorrichtung zum Inkontaktbringen (7) bei einer Temperatur von weniger als 50 °C verdampft wird,
wobei das mindestens eine Pflanzenschutzmittel ausgewählt ist aus der folgenden Liste: ätherisches Öl, Terpene, gesättigter oder ungesättigter C3-C9-Alkohol, Glutaraldehyd, Hexanal, Dimethylnaphthalin und 3-Decen-2-on; wobei das mindestens eine Biozidmittel ausgewählt ist aus der folgenden Liste: Nelkenöl, Thymianöl, Geraniol, Ethylalkohol, Glutaraldehyd.

7. Anordnung nach Anspruch 6, **dadurch gekennzeichnet, dass** die Anordnung eine Vorrichtung (95) zum Regenerieren des Flüssigkeitsstroms umfasst, wobei:
- eine äußere Vorrichtung (97) zum Inkontaktbringen, die eine äußere Packung (99) umfasst;
- eine Vorrichtung (101) zum Einspritzen des Flüssigkeitsstroms aus der Vorrichtung zum Inkontaktbringen (31) in die äußere Vorrichtung zum Inkontaktbringen (95);
- eine Vorrichtung (103) zur Zirkulation eines Luftstroms von außerhalb des Lagers (3) in die äußere Vorrichtung zum Inkontaktbringen (95);
wobei die äußere Kontaktvorrichtung (95) ausgebildet ist, damit der Flüssigkeitsstrom durch Zirkulation in der äußeren Packung (99) mit einem Luftstrom außerhalb des Lagers (3) in Kontakt gebracht wird und dann in die Vorrichtung zum Inkontaktbringen (31) zurückgeführt wird.

## Claims

1. A method for treating the atmosphere of a storage (3) of vegetable products (5) having a volume of more than 1000 m³, the method comprising at least a step of contacting the atmosphere with a liquid flow by circulation in a packed bed, the liquid comprises at least one volatile biocidal and/or herbicide safener product, with a boiling temperature comprised between 60 and 280°C, the liquid being evaporated in the contacting step at a temperature of less than 50°C; the at least one herbicide safener products being selected from the following list: essential oil, terpenes, saturated or unsaturated C₃ - C₉ alcohol, glutaraldehyde, hexanal, dimethylnaphthalene and 3-decene-2-one; the at least one biocidal product being selected from the following list: clove oil, thyme oil, geraniol, ethyl alcohol, glutaraldehyde.

2. The method according to any of the claim 1, **characterized in that** the method comprises:
- a step for collecting the liquid flow from the contacting step;
- a step for regenerating the liquid flow stemming from the contacting step, during which said liquid flow is put into contact with an air flow outside the storage by circulation in an outer packed bed, the liquid flow then being reused in the contacting step.

3. The method according to claim 2, **characterized in that** the method comprises a step for measuring the CO₂ and/or O₂ concentration in the atmosphere of the storage, a flow rate of the liquid flow being adjusted according to said measured concentration.

4. The method according to claim 2 or 3, **characterized in that** the method comprises a step for measuring the temperature and/or the humidity in the atmosphere of the storage, a flow rate of the atmosphere in the contacting step being adjusted according to said measured temperature and/or humidity.

5. The method according to any of claims 2 to 4, **characterized in that** the method comprises a step for measuring the temperature in the atmosphere of the storage, a flow rate of the air flow outside the regeneration step being adjusted at least according to said measured temperature.

6. An assembly for treating the atmosphere of a storage (3) of vegetable products (5) having a volume greater than 1000 m³, the assembly (1) comprising at least:
- a contacting device (7, 31), including a packed bed (9, 33);
- a device (11, 35) for injecting a liquid flow into the contacting device (7, 31);
- a device (13, 36) for circulating the atmosphere of the storage (3) in the contacting device (7, 31);
the contacting device (7, 31) being conformed so that the atmosphere is put into contact with the liquid flow by circulation in the packed bed (9, 33),
the contacting device (7) includes a reserve (15) and a dose of liquid stored in the reserve, the liquid comprising at least one volatile biocidal and/or herbicide safener product with a boiling temperature comprised between 60 and 280°C, the liquid being evaporated in the contacting device (7) at a temperature of less than 50°C,
the at least one herbicide safener products being selected from the following list: essential oil, terpenes, saturated or unsaturated C₃ - C₉ alcohol, glutaraldehyde, hexanal, dimethylnaphthalene and 3-decene-2-one; the at least one biocidal product being selected from the following list: clove oil, thyme oil, geraniol, ethyl alcohol, glutaraldehyde.

7. The assembly according to claim 6, **characterized in that** the assembly comprises a device (95) for regenerating the liquid flow, with:
- an outer contacting device (97), including an outer packed bed (99);
- a device (101) for injecting the liquid flow from the contacting device (31) in the outer contacting device (95);
- a device (103) for circulating an air flow outside the storage (3) into the outer contacting device (95);
the outer contacting device (95) being conformed so that the liquid flow is put into contact with an air flow outside the storage (3) by circulation in the outer packed bed (99), and then recycled in the contacting device (31).
